# EUROPEAN PATENT SPECIFICATION

(11) **EP 1 240 307 B1**
(45) Date of publication and mention of the grant of the patent: **15.09.2004**
(21) Application number: 00979881.0
(22) Date of filing: 18.12.2000
(51) Int. Cl.: C12M 1/12

(54) **DEVICE FOR MICROBIOLOGICAL EXAMINATION OF A SAMPLE OF LIQUID AND METHOD FOR DRAINING THIS DEVICE**
EINHEIT FÜR MIKROBIOLOGISCHE PRÜFUNG EINER FLÜSSIGEN PROBE UND VERFAHREN FÜR DAS LEEREN DIESER EINHEIT
DISPOSITIF SERVANT A EFFECTUER L'EXAMEN MICROBIOLOGIQUE D'UNE ECHANTILLON LIQUIDE ET PROCEDE DE DRAINAGE DE CE DISPOSITIF

(30) Priority: 24.12.1999 FR 9916459
(43) Date of publication of application: 18.09.2002
(73) Proprietor: MILLIPORE, 67120 Molsheim (FR)
(72) Inventor: LEMONNIER, Jean, F-75006 Paris (FR)
(74) Representative: Santarelli
(86) International application number: PCT/IB2000/001908
(87) International publication number: WO 2001/048142

(56) References cited:
- EP-A- 0 059 809
- EP-A- 0 319 701
- US-A- 4 319 996
- US-A- 4 678 576

## Description

The invention relates to the devices for microbiological examination of a sample of liquid.

Such devices are already known, which have an intake body, a filtering membrane and a drainage body designed to support the membrane on the opposite side from the intake body.

In general, these devices work by simple gravity and have the general form of a funnel into which the sample to be examined is introduced through the upper opening while the liquid which has passed through the membrane is recovered by means of the output pipe. There also exists such a device provided for the sampling of a liquid under pressure, described in French patent 2 677 664.

The invention aims to make it possible to culture micro-organisms collected by filtration on the membrane under the best possible conditions.

To that end it proposes a device for microbiological examination of a sample of liquid, having an intake body, a filtering membrane and a drainage body having means of supporting said membrane on the opposite side from said intake body; characterised in that said support means have a concave surface facing said membrane, and in that the ratio of the difference between the length of the arc corresponding to the profile, in a diametral plane, of said surface of said support means and between the length of the chord of this arc, over the latter length, corresponds to the coefficient of expansion of said membrane between the dry state and the wet state.

By virtue of the concave nature of this surface, it is possible to avoid the creases which form on the membrane of the earlier devices, where the corresponding surface is flat, on account of the expansion which the membrane undergoes when it changes from the dry state to the wet state.

Moreover, once the sampling has been performed, the membrane has a concave form on the intake side, that is to say on the side where any retained micro-organisms are present, the curvature of the membrane thus being in the correct direction where putting it down on the surface of the culture medium in the Petri dish is concerned, minimizing the risks of trapping one or more pocket(s) of air between the culture medium and the membrane, which are particularly dangerous since they can lead to false results, and in particular to the conclusion that the sample meets health standards whereas in reality the absence or the small quantity of micro-organisms on the membrane after incubation results from the fact that the membrane was isolated from the culture medium by the pocket or pockets of air.

The difference ratio makes it possible to achieve the best cooperation between the membrane and the support means.

It should be noted that filtration units comprising an intake body, a filtering membrane and a drainage body having means of supporting the membrane on the opposite side from the intake body having a concave surface facing the membrane was already known outside the field of devices for microbiological examination of a sample of liquid, in particular from US 4 319 116 A and from EP 0 319 701 A. These filtration units can not be used for microbiological examination of a sample of liquid, because they are not designed for being opened after filtering the liquid so as to recover the membrane. The reason for which a concave surface is provided in these filtration units is exclusively the maintenance of the filtration capacities, by prevention of blocking respectively because of an air bubble or because of clogging. In the devices disclosed by these documents, it is merely stated that the filter is deformable and that the required deformation for taking the concave shape of the bearing surface is less than the deformation that would damage the filter (there is no question of the expansion coefficient of a membrane between the dry state and the wet state).

According to preferred characteristics, for reasons of simplicity and convenience, both in manufacture and in use, said support means are formed by a porous pad.

According to other preferred characteristics, said drainage body has an output aperture in the continuation of the internal passage of a coaxially disposed output pipe with preferably the drainage body having, around said output pipe, an annular rib tapering towards its end.

It is then possible to drain the device as described above by placing it on a vacuum flask with said output pipe engaged in the central hole of the stopper of said flask and said annular rib resting on this stopper.

The invention also relates, in a second aspect, to this drainage method.

It should be noted that it is also possible to use this method and provide the arrangement described above of the output pipe and the annular rib of the drainage body, in any device for microbiological examination of a liquid, including one with a means of supporting the membrane whose surface is flat.

The explanation of the invention will now be continued with the description of an example embodiment, given below as a non-limitative illustration, with reference to the accompanying drawings. In these:
- Figure 1 is an elevational view of a device in accordance with the invention;
- Figure 2 is a sectional elevational view of this device;
- Figures 3 and 4 are similar views but showing, respectively, only the intake body and the drainage body;
- Figure 5 is an enlargement of the part of Figure 2 situated at the bottom right;
- Figure 6 is a partial sectional elevational view of the seal with which the intake body is provided;
- Figure 7 is a sectional elevational view showing how the device according to the invention is used for sampling the liquid to be examined;
- Figure 8 is a similar view showing how the device in accordance with, the invention is drained, after a sample has been taken, by means of a syringe;
- Figure 9 is the corresponding top view, a second possible location for the syringe being shown with a syringe partially illustrated;
- Figure 10 is a view similar to Figure 8, where the drainage is performed with a vacuum flask;
- Figures 11 and 12 are sectional elevational views showing how the latching tabs are broken off the intake body in order to release the latter from the drainage body;
- Figure 13 shows how the membrane is recovered with tweezers after this release; and
- Figure 14 shows how the membrane is deposited in a Petri dish.

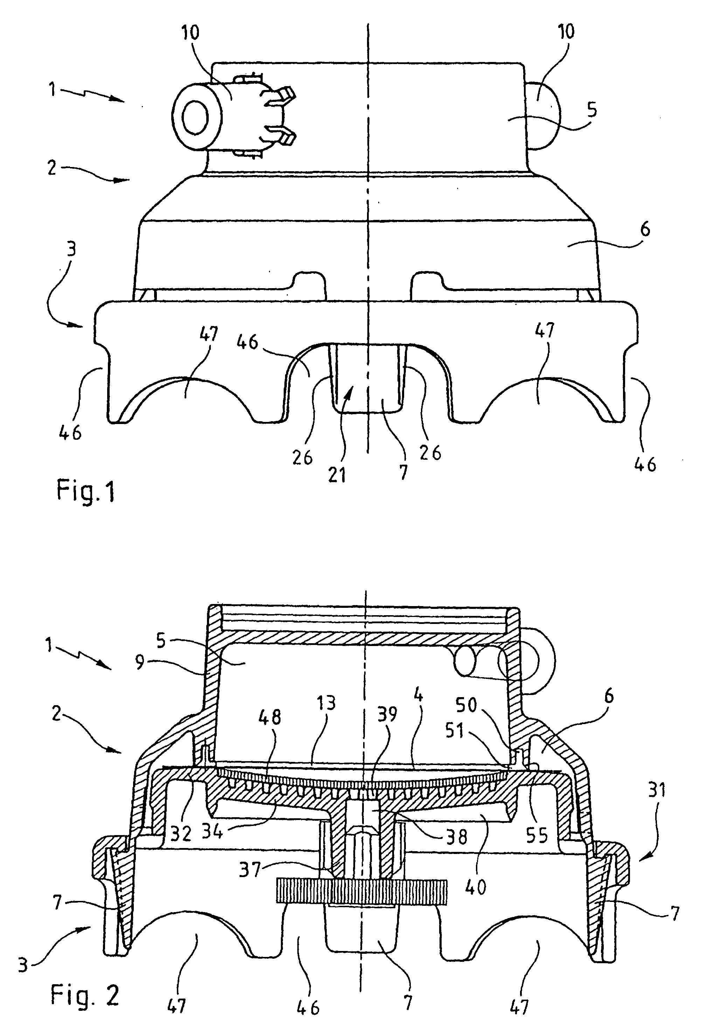

The device 1 for microbiological examination of a sample of liquid under pressure shown in the drawings, and notably in Figures 1 and 2, has in general terms a symmetry of revolution around a central axis. It has an intake body 2, a drainage body 3 and a filtering membrane 4.

The intake body 2 has a reservoir 5, a skirt 6 which is connected externally to the reservoir 5 and four latching tabs 7 which extend projecting from the skirt 6, in an axial direction.

The reservoir 5 has an end wall 8 and a lateral wall 9.

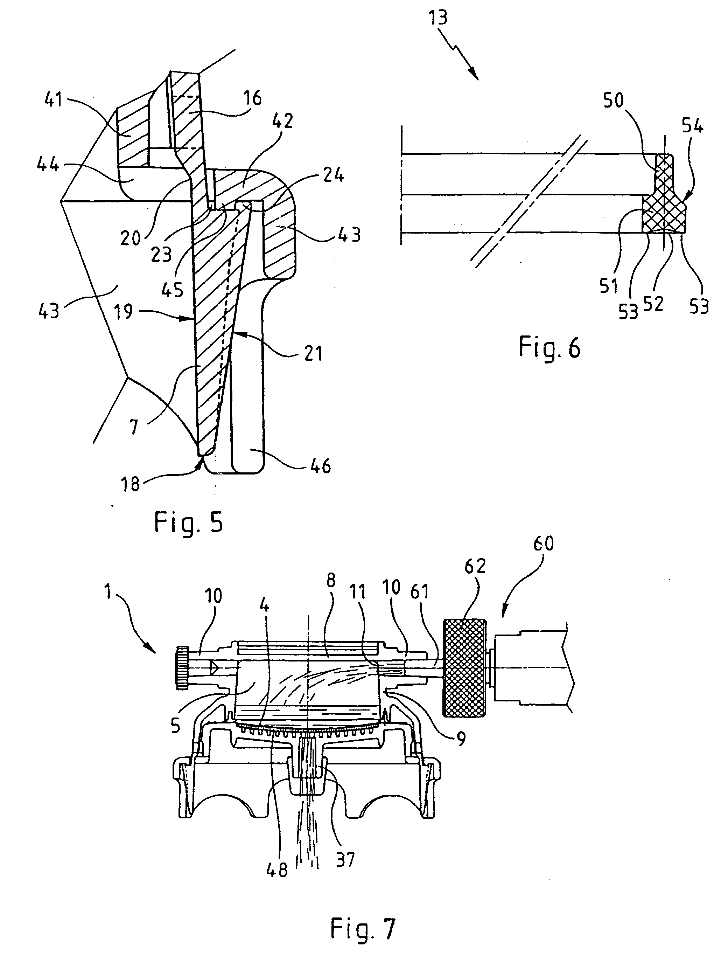

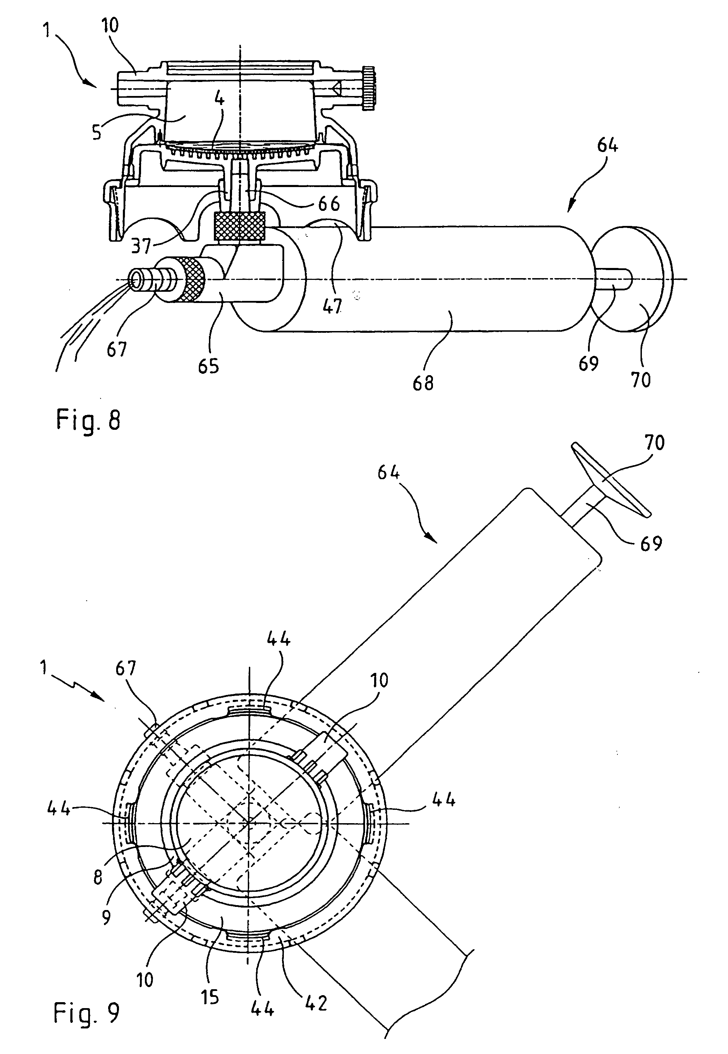

Two diametrically opposite pipes 10 extend projecting outward from the lateral wall 9, above the skirt 6, each of these pipes constituting a female Luer connector adapted to receive internally a male Luer connector, as will be explained below with the help of Figure 7, the passage internal to each pipe 10 being continued by an aperture 11 made in the wall 9, this aperture being in immediate proximity to the end wall 8.

The lateral wall 9 finishes at the end opposite the end wall 8 in an edge forming part of a seal 13, a groove 14 being made to that effect in the rigid part of the wall 9, as will be explained in more detail subsequently with the help of Figures 2, 3 and 6.

The skirt 6 is connected to the reservoir 5 by the outside of the lateral wall 9, at a level situated between the groove 14 and the pipes 10, the skirt 6 having a truncated-cone shaped wall 15 and a cylindrical wall 16, the skirt 6 being connected to the wall 9 by the small-diameter end of the wall 15 while the connection between the walls 15 and 16 is made by the large-diameter end of the wall 15, the connection between the walls 15 and 16 being situated approximately at the level of the edge of the wall 9.

Each of the latching tabs 7 has a general outline in the form of a trapezium symmetrical with respect to the axial direction, the side forming the free end 18 of the tab 7 being parallel to the one by which this tab is connected to the skirt 6, and more precisely to the edge of the wall 16, the tab 7 narrowing steadily between its connection to the skirt 6 and its free end.

On either side of each tab 7, a notch 17 is made in the wall 16, over a certain distance from the edge thereof.

Each tab 7 has, from its free end 18, an internal surface 19 which is straight, that is to say parallel to the axial direction, as far as a dihedral 20 from which the surface 19 is inclined inward and towards the wall 16.

As for the external surface 21 of each tab 16, this is inclined outward and towards the wall 16, the surface 21 extending between the surface 18 and a transversely oriented surface 22 which connects the surface 21 and a groove 23 situated between an external shoulder 24 whose surface 22 constitutes the edge and a surface 25 offset inward with respect to the surface 21, the surface 25 being in the continuation of the external surface of the wall 16.

It should be noted that the portion of each tab 7 situated between the bottom of the groove 23 and the edge of the wall 16 has a thickness which is a minimum at the level of the dihedral 20.

Consequently, it is in the region of the dihedral 20 that the tab 7 breaks if a sufficiently large pressure is exerted on the surface 21, and more generally if there is exerted on the tab 7 a radial force directed inward, the force necessary for breaking the tab 7 being smaller the closer it is applied to the end surface 18.

As can be seen more particularly in Figure 1, the surface 21 has edges parallel to the axial direction, each tab 7 having a notch 26 with an L-shaped profile between the lateral edges of the surface 21 and the lateral edges of the tab 7.

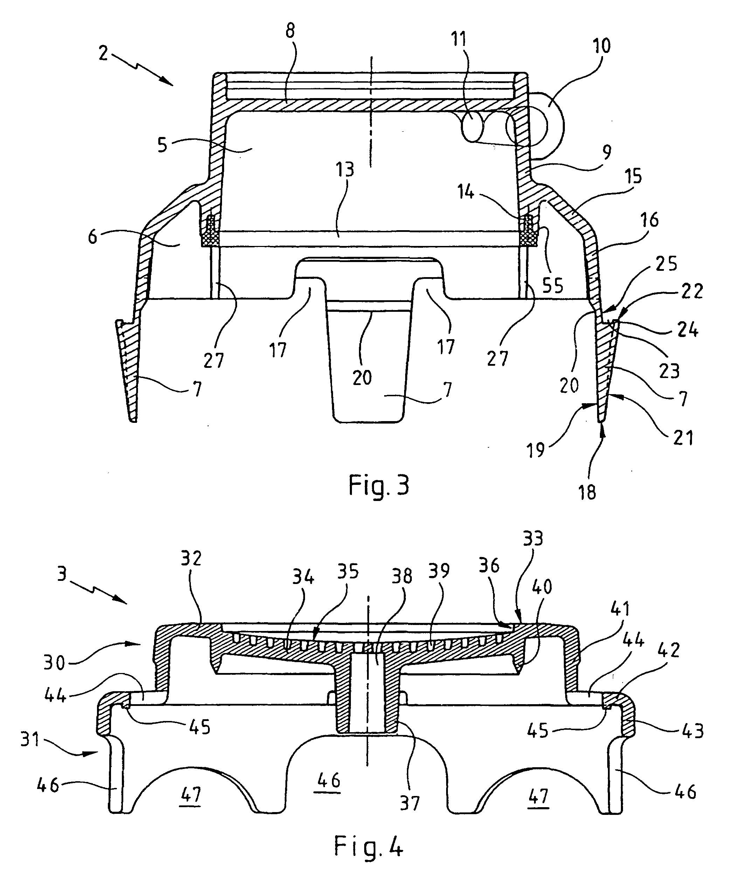

As can be seen better in Figure 4, the drainage body 3 has a circular table 30 and a skirt 31 disposed in a step around the table 30.

The latter has an annular transverse wall 32 delimited on the opposite side from the skirt 31 by a surface 33 which is flat in the main but having a slight bevel towards the outside.

The internal periphery of the wall 32 is connected to a wall 34 delimited, on the side of the surface 33, by a surface 35 which is concave in the main, offset with respect to the surface 32 in the axial direction, towards the skirt 31, the perimeter of the surface 35 and the internal periphery of the surface 33 being connected by a slightly truncated-cone shaped surface 36.

The wall 34 is connected centrally to a pipe 37 whose internal passage is extended into the wall 34 by an output aperture 38, concentric drainage channels 39 being put into the wall 34 from the surface 35, radially oriented channels (not visible in the drawings) also being made, with the same depth as the channels 39, these radial channels opening of course into the output aperture 38, through which, therefore, there flows out all the liquid drained by the channels made in the wall 34 hollowed out with respect to the surface 35.

At the junction between the walls 32 and 34 there is situated an annular rib 40 which projects with respect to the walls 32 and 34 on the side of the skirt 31, this rib tapering towards its free end in a V-shaped profile, so that this end constitutes a sharp edge.

The table 30 also has a tubular lateral wall 41 which is connected by one end to the wall 32 while, by the other end, it is connected to the skirt 31.

The latter has a transversely oriented annular wall 42 and an axially oriented cylindrical wall 43, the wall 42 being connected by one of its ends to the wall 41 and by the other to the wall 43.

In the wall 42, in proximity to the wall 41, four openings 44 are made, which have between them the same angular spacing as between the latching tabs 7, that is to say they are spaced out from one another by 90°, these openings having an outline corresponding to the largest outline of the tabs 7, so that the latter can each pass through a respective opening 44.

Each opening 44 is bordered on the external side by an axially oriented tooth 45 projecting on the opposite side from the table 30.

Each tooth 45 extends projecting over a height corresponding to the depth of the groove 23 and has a thickness less than the width of the groove 23, the distance separating each tooth 45 from the wall 43 being greater than the thickness of the shoulder 24 (see Figure 5).

At the level of each opening 44, the wall 43 has a notch 46 of general rectangular form with rounded corners, extending over approximately two thirds of the height of the wall 43 and over a width which is approximately twice the width of the latching tabs 7.

The wall 43 also has four notches 47, each disposed halfway between two successive notches 46, the notches 47 having a rounded form whose maximum height corresponds approximately to one third of the height of the wall 43.

The drainage body 3 also has a porous pad 48 (not depicted in Figure 4), which has a constant thickness with two opposite surfaces of the same form as the surface 35, its diameter and thickness being the same as those of the surface 36.

When the filtration body 2, the drainage body 3 and the membrane 4 are assembled, as shown notably in Figures 1 and 2, the membrane 4 is gripped between the edge of the lateral wall 9 of the reservoir 5 of the intake body 2 and the surface 33 of the wall 32 of the circular table 30 of the drainage body 3, the bodies 2 and 3 being locked to one another by virtue of the latching tabs 7 and the skirt 31, which are mutually disposed as can be seen more particularly in Figure 5.

It should be noted that the tooth 45 of the wall 42 fits into the groove 23 of the tab 7 and that the shoulder 24 of this tab fits into the space situated between the wall 43 and the tooth 45, so that the cooperation between the shoulder 24 and the tooth 45 provides an extremely powerful locking of the tab 7 in the skirt 31, capable of withstanding relatively large forces tending to move the bodies 2 and 3 away from one another.

It should also be noted that the end 18 of the tab 7 is recessed with respect to the free end of the wall 43, so that, when the device 1 is put down on a surface with the drainage body 3 at the bottom, it is by means of the skirt 31 thereof that the device 1 rests on this surface, no force being exerted for this reason on the tabs 7, which therefore do not risk being broken accidentally.

As can be seen in Figure 2, when the device 1 is assembled, the seal 13, and more particularly the cushion thereof, is highly compressed compared with the off-load form of this seal shown in Figure 6.

As indicated above, this seal has a T-shaped general profile whose longitudinal branch forms a rib 50 designed to be inserted into the groove 14 and whose transverse branch forms a cushion 51 designed to enter into contact with the membrane 4.

The free end of the cushion 51 has a central slot 52 which makes it possible to release two annular lips 53 allowing the best cooperation of the cushion 51 with the membrane 4.

It should be noted that the junction between the rib 50 and the cushion 51 is made by a straight surface on the internal side while, on the external side, there is a bevel 54.

This bevel in fact corresponds to a chamfered lip 55 at the external periphery of the end of the rigid part of the wall 9, this chamfered lip making it possible to laterally contain the cushion 51 on the external side in order that it flows mainly inward, that is to say towards the chamber delimited by the membrane 4 and the reservoir 5.

The intake body 2 is obtained, with the exception of the seal 13, by moulding of a relatively rigid and transparent plastic, and then there is moulded, on to this piece, the seal 13, which is made of elastomer, this over-moulding being carried out in the example illustrated by bi-injection.

The part of the drainage body 3 depicted in Figure 4 is also made of relatively rigid moulded plastic, here white in colour, this part being next equipped, by simple fitting, with the porous pad 48.

In order to assemble the intake body 1, the drainage body 3 and the membrane 4, the latter is put on the table 30, concentrically therewith, then the intake body 2 is positioned facing the drainage body 3 with the latching tabs 7 aligned with the openings 44, then the body 2 is pressed hard towards the body 3 so that the tabs 7 engage in the openings 44 flexing slightly by virtue of the inclined surface 21 which acts as a ramp, the force exerted allowing the surface 22 of the shoulder 24 to get over the tooth 45 at the end of the pushing in movement, by virtue of the spring of the tabs 7, the seal 13 next relaxing slightly so that the play between the tabs 7 and the skirt 31 is completely taken up, the elasticity of the seal 13, which is then compressed, maintaining the locking thus obtained.

It should be noted that the maintaining of the seal in the compressed state allows it to offer excellent sealing between the membrane 4 and the edge of the wall 9, and furthermore, by reaction, between the membrane 4 and the surface 33.

It should also be noted that the internal surface of the wall 16 has localized areas of extra thickness 27 (Figure 3) coming into contact with the external surface of the wall 41, which provides a lateral wedging between these surfaces, which are of similar diameter, and more generally between the bodies 2 and 3.

Finally it should be noted that, once the device 1 has been assembled in this way, it is possible to package it and sterilize it with a gas such as ETO or by irradiation.

Of course, before packaging the assembled device 1 and sterilizing it, each of the pipes 10 and 37 is equipped with a stopper.

There will now be explained how the sampling of a liquid under pressure is carried out with the device 1.

First of all the stopper blocking off one of the pipes 10 and the stopper blocking off the pipe 37 are removed, then the unstoppered pipe 10 is connected to a source of liquid under pressure, for example using, as shown in Figure 7, a sampling connector 60 having a male Luer tip 61, which is inserted into the passage of the unstoppered pipe 10 and the valve 62 of the connector 60 is manipulated, so that the chamber formed by the reservoir 5 and the membrane 4 is raised to the same pressure as the liquid, for example 3 bars, the liquid entering the reservoir 5 through the aperture 11 and leaving the reservoir by passing through the membrane 4, which comes to rest on the porous pad 48, the liquid which has passed through the membrane 4 being guided by the channels 39 to the aperture 38, the liquid leaving the device 1 by the pipe 37, a graduated container being preferably disposed under the device 1 in order to recover the liquid coming out of the pipe 37 in order to know when the volume required for the sample has passed through the membrane 4.

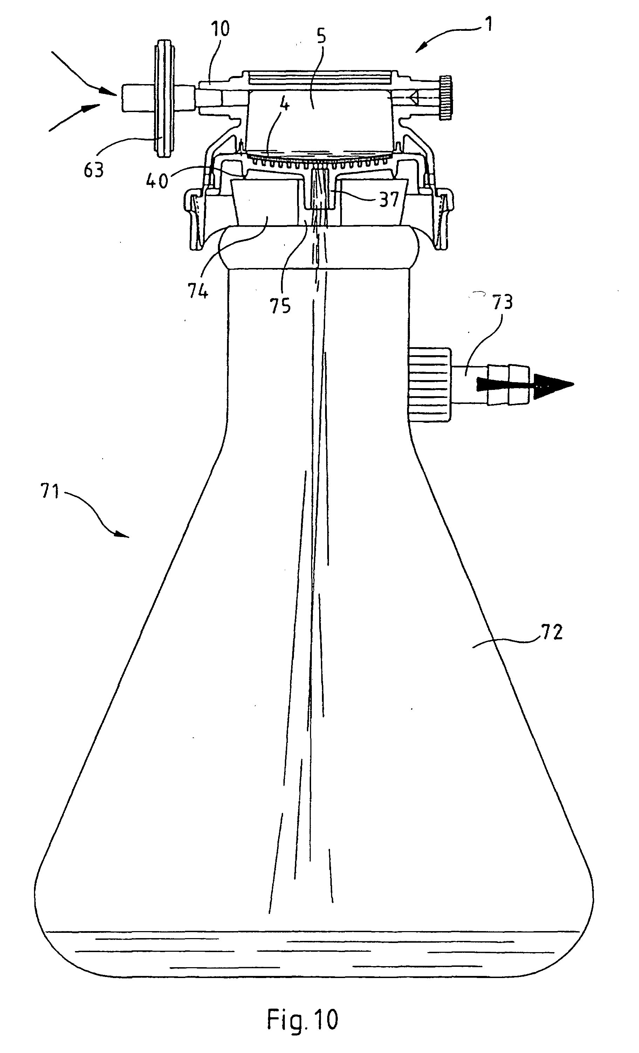

When this volume has been reached, the valve 62 is closed and the device 1 is removed from the connector 60, then there is put in place, in the unstoppered pipe 10, an air sterilization filter 63 (depicted in Figure 10 but not in Figure 8), and the drainage of the liquid still present notably in the reservoir 5 is next carried out, by suction through the output aperture 38.

In the example shown in Figure 8, the drainage is performed with a syringe or pump 64 having a connector 65 provided with a suction tip 66 which has been inserted into the passage of the pipe 37, the liquid sucked out by the tip 66 being expelled by the tip 67 when the shaft 69 is pushed into the body 68, by pressing on the plunger 70.

It should be noted that the notches 47 made in the wall 43 make it possible to correctly position the pump or syringe 64 in relation to the device 1, in four positions at 90° from one another, two of these positions being shown in Figure 8.

Another possibility for extracting the liquid remaining in the device 1 after sampling, is to use a vacuum flask, as shown in Figure 10.

The vacuum flask 71 illustrated has a glass body 72 having, at the level of its neck, a pipe 73 connected, in a manner not depicted, to a vacuum pump, and, at the top of this neck, a flexible stopper 74 with a central aperture 75 made in it, the flask 71 being of a type which is commonly found in practice.

The device 1 is simply put down on the stopper 74, with the pipe 37 engaged in the aperture 75 and the rib 40 supported on the top of the stopper 74.

On account of the tapered profile of the rib 40, the latter locally deforms the stopper 74 and provides sealing which makes it possible to suck out the residual liquid, as drawn.

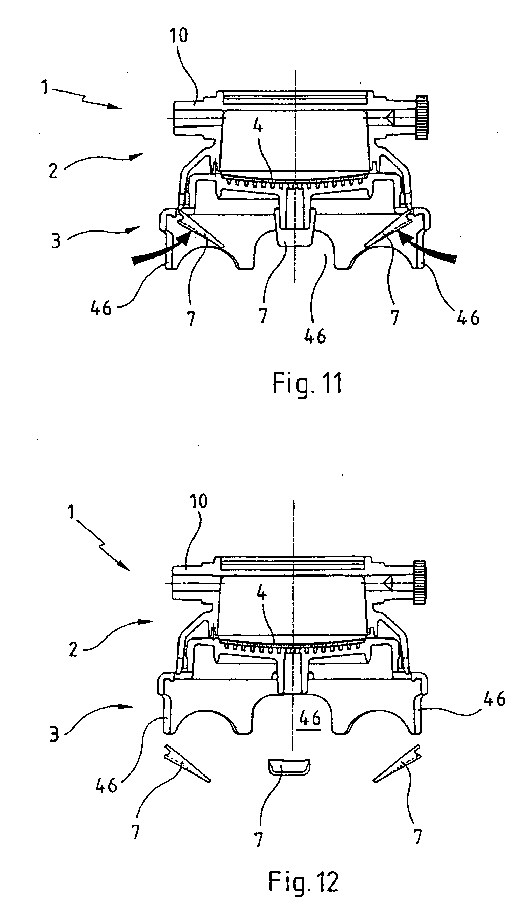

Once the liquid remaining in the device 1 has been emptied therefrom, the device 1 can be opened, which is performed by breaking the four latching tabs 7, by simple pressure on said tabs through the respective notches 46, as explained above and illustrated in Figures 11 and 12.

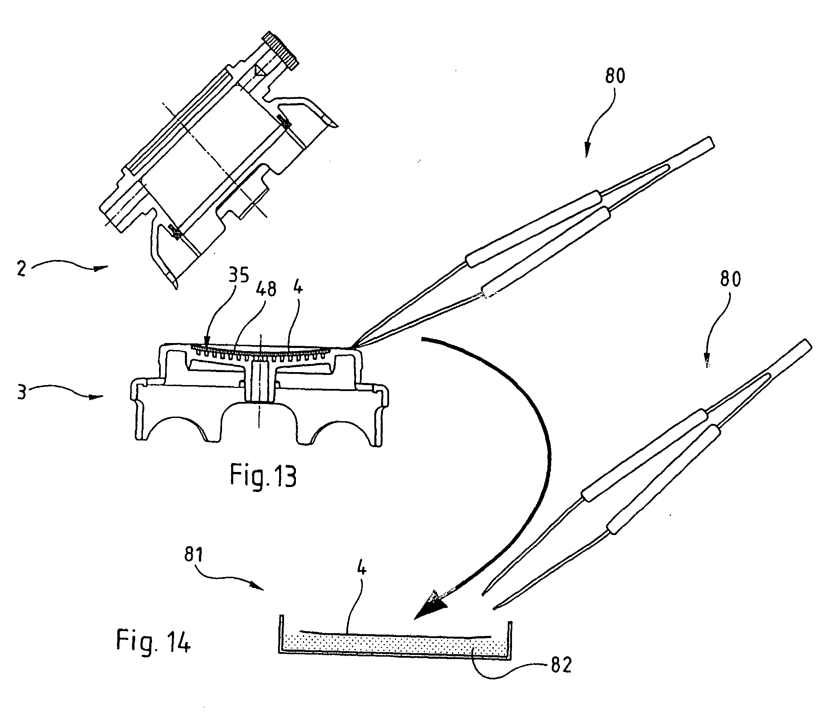

It is then possible to remove the intake body 2 from the drainage body 3 and pick up the membrane 4, for example with sterile tweezers 80, as shown in Figure 13, then deposit the membrane through which the sample to be examined has passed, in a Petri dish 81, as shown in Figure 14, then carry out conventionally the incubation of the membrane/Petri dish assembly.

It should be noted that the concavity of the surface 35 has been calculated so that the ratio of the difference between the length of the arc corresponding to the profile, in a diametral plane, of the surface of the pad 48 facing the membrane 4 and between the length of the chord of this arc, over the latter length, corresponds to the coefficient of expansion of the membrane 4 between the dry state and the wet state.

The result thereof is that the expansion of the membrane 4, when it changes from the dry state to the wet state, corresponds precisely to the difference in length between the arc corresponding to the above-mentioned profile and the chord of this arc, so that, in the wet state, the membrane 4 rests perfectly on the pad 48, with no creases. The pad 48 therefore provides a particularly effective support for the membrane 4 when it is subjected to the difference in pressure which allows the liquid to flow through it.

Moreover, when the user recovers the membrane 4 with the tweezers 80 as shown in Figure 13, this membrane has a concave form, on the side where the reservoir 5 is situated, that is to say on the side where any micro-organisms retained by the membrane at the time of sampling are present, the curvature of the membrane 4 thus being in the correct direction where putting it down on the surface of the culture medium 82 in the dish 81 is concerned.

This is because, when the membrane 4 is positioned on the dish 81, it is the convex side of the membrane 4 which faces the surface of the medium 82, so that, putting down the membrane 4 on the medium 82 from a portion of the membrane opposite the tweezers 80 and moving them so that the membrane progressively comes into contact with the medium 82 to the place where it is held by the tweezers. The risk that the membrane has one or more hollows on the opposite side from the medium 82, and therefore the risk that it develops one or more pocket(s) of air between the membrane 4 and the medium 82, are thus zero or at any rate minimal.

The culture medium 82 in the dish 81 illustrated in Figure 14 is a culture medium containing agar-agar, used in the solid state after having been poured into the dish hot.

If it is wished to use a liquid culture medium, it is possible to replace the Petri dish 81 with a similar dish but one where the agar-agar culture medium 82 is replaced by an absorbent pad impregnated with liquid culture medium.

Another possibility, rather than culturing the micro-organisms outside the device 1, is to inject liquid culture medium therein using one of the pipes 10, then to drain the excess culture medium using the pipe 37, and to next put the device 1 to incubate directly, the membrane 4 being recovered only in order to identify and count the micro-organisms after incubation.

In such a case, there is an advantage in using a liquid culture medium which is slightly more concentrated than the conventional media since there always remains, notably in the pad 48, a certain amount of the sampled liquid which mixes with the injected culture medium which is therefore diluted.

In a variant. not depicted, the device according to the invention is designed to work by gravity, and therefore has a different intake body.

Many other variants are possible depending on circumstances, and it should be stated in this respect that the invention is not limited to the examples described and depicted.

## Claims

1. Device for microbiological examination of a sample of liquid, having an intake body, a filtering membrane and a drainage body having means of supporting said membrane on the opposite side from said intake body; **characterised in that** said support means (48) have a concave surface facing said membrane (4), and **in that** the ratio of the difference between the length of the arc corresponding to the profile, in a diametral plane, of said surface of said support means (48) and between the length of the chord of this arc, over the latter length, corresponds to the coefficient of expansion of said membrane (4) between the dry state and the wet state.

2. Device according to Claim 1, **characterised in that** said support means are formed by a porous pad (48).

3. Device according to Claim 2, **characterised in that** said drainage body (3) has drainage channels (39) under said porous pad (48), said drainage channels opening into an output aperture (38).

4. Device according to any one of Claims 1 to 3, **characterised in that** said drainage body (3) has a circular table (30) provided at its centre with said means of supporting (48) said membrane (4) and having, around said support means (48), a wall (32) having a surface (33) situated facing said elastomer seal (13), which forms part of said intake body (2), said membrane (4) being squeezed between said surface (33) and said seal (13).

5. Device according to Claim 4, **characterised in that** the external diameter of said circular table (30) corresponds substantially to the internal diameter of a skirt (6) included in said intake body (2), said skirt (6) encircling said circular table (30).

6. Device according to Claim 5, **characterised in that** areas of extra thickness for wedging (27) are provided between said circular table (30) and said skirt (6).

7. Device according to any one of Claims 4 to 6, **characterised in that** said drainage body has a skirt (31) disposed in a step with respect to said circular table (30).

8. Device according to Claim 7, **characterised in that** said skirt (31) has means of latching (42, 44, 45) with said intake body (2).

9. Device according to either one of Claims 7 or 8, **characterised in that** said skirt (31) of the drainage body (3) has at least one notch (47) adapted to allow the placing of a drainage syringe (64).

10. Device according to any one of Claims 1 to 9, **characterised in that** said drainage body (3) has an output aperture (38) in the continuation of the internal passage of a coaxially disposed output pipe (37).

11. Device according to Claim 10, **characterised in that** said drainage body (3) has, around said output pipe (37), an annular rib (40) tapering towards its end.

12. Method for draining a device according to Claim 11, **characterised in that** it is placed on a vacuum flask (71) with said output pipe (37) engaged in the central hole (75) of the stopper (74) of said flask and said annular rib (40) resting on this stopper.

## Patentansprüche

1. Vorrichtung für mikrobiologische Untersuchung einer Flüssigkeitsprobe mit einem Einlasskörper, einer Filtermembran und einem Drainagekörper mit Mitteln zum Halten bzw. Abstützen der Membran an der dem Einlasskörper gegenüberliegenden Seite,
**dadurch gekennzeichnet, dass** die Halte- bzw. Abstützmittel (48), eine konkave Oberfläche besitzen, die der Membran (4) zugewandt ist, und dadurch, dass das Verhältnis der Differenz zwischen der Länge des Bogens entsprechend dem Profil der Oberfläche der Haltemittel (48) in einer diametralen Ebene, und der Länge der Sehne dieses Bogens gegenüber der letzteren Länge dem Expansionskoeffizienten der Membran (4) zwischen dem trockenen Zustand und dem nassen Zustand entspricht.

2. Vorrichtung gemäß Anspruch 1, **dadurch gekennzeichnet, dass** die Haltemittel durch eine poröse Unterlage (48) gebildet werden.

3. Vorrichtung gemäß Anspruch 2, **dadurch gekennzeichnet, dass** der Drainagekörper (3) Drainagekanäle (39) unter der porösen Unterlage (48) aufweist, wobei sich die Drainagekanäle in eine Auslassöffnung (38) öffnen.

4. Vorrichtung gemäß einem der Ansprüche 1 bis 3, **dadurch gekennzeichnet, dass** der Drainagekörper (3) einen kreisförmigen Tisch (30) besitzt, der in seiner Mitte mit den Halte- bzw. Abstützmitteln (48) der Membran (4) versehen ist und um die Halte- bzw. Abstützmittel (48) herum eine Wand (32) mit einer Oberfläche (33) aufweist, die der Elastomerdichtung (13) zugewandt gelegen ist, welche einen Einlaßkörpers (2) bildet, wobei die Membran (4) zwischen der Oberfläche (33) und der Dichtung (13) gedrückt ist.

5. Vorrichtung gemäß Anspruch 4, **dadurch gekennzeichnet, dass** der Außendurchmesser des kreisförmigen Tisches (30) im wesentlichen dem Innendurchmesser eines in dem Einlasskörper (2) enthaltenen Randteils (6) entspricht, wobei das Randteil (6) den kreisförmigen Tisch (30) umgibt.

6. Vorrichtung gemäß Anspruch 5, **dadurch gekennzeichnet, dass** Bereiche mit zusätzlicher Dicke bzw. Stärke für eine Verkeilung (27) zwischen dem kreisförmigen Tisch (30) und dem Randteil (6) vorgesehen sind.

7. Vorrichtung gemäß einem der Ansprüche 4 bis 6, **dadurch gekennzeichnet, dass** der Drainagekörper einen Randteil (31) besitzt, der in einer Abstufung bezüglich dem kreisförmigen Tisch (30) angeordnet ist.

8. Vorrichtung gemäß Anspruch 7, **dadurch gekennzeichnet, dass** das Randteil (31) Mittel zum Verriegeln (42,44,45) mit dem Einlasskörper (2) aufweist.

9. Vorrichtung gemäß einem der Ansprüche 7 oder 8, **dadurch gekennzeichnet, dass** das Randteil (31) des Drainagekörpers (3) mindestens eine Einkerbung (47) besitzt, die das Ansetzen einer Drainagespritze (64) zulässt.

10. Vorrichtung gemäß einem der Ansprüche 1 bis 9, **dadurch gekennzeichnet, dass** der Drainagekörper (3) eine Auslassöffnung (38) in der Fortsetzung des internen Durchgangs eines axial angeordneten Auslassrohrs (37) besitzt.

11. Vorrichtung gemäß Anspruch 10, **dadurch gekennzeichnet, dass** der Drainagekörper (3) um das Auslassrohr (37) herum eine ringförmige Rippe (40) besitzt, die zu ihrem Ende hin schräg verläuft.

12. Verfahren zum Entwässern bzw. Entleeren einer Vorrichtung gemäß Anspruch 11, **dadurch gekennzeichnet, dass** sie auf einen Vakuumkolben bzw. eine Vakuumflasche oder einen Vakuumbehälter (71) aufgesetzt wird, wobei das Auslassrohr (37) in Eingriff in das Mittelloch (75) des Stopfens (74) des Kolbens/Behälters/der Flasche eingreift und die ringförmige Rippe (40) auf diesem Stopfen aufliegt.

## Revendications

1. Dispositif pour le contrôle microbiologique d'un échantillon de liquide, ayant un corps d'admission, une membrane filtrante et un corps de drainage comportant des moyens de support de ladite membrane sur le côté opposé par rapport audit corps d'admission ; **caractérisé en ce que** lesdits moyens de support (48) ont une surface concave faisant face à ladite membrane (4), et **en ce que** le rapport de la différence entre la longueur de l'arc correspondant au profil, dans un plan diamétral, de ladite surface desdits moyens de support (48) et la longueur de la corde de cet arc, sur la longueur de ce dernier, correspond au coefficient de dilatation de ladite membrane (4) entre l'état sec et l'état mouillé.

2. Dispositif selon la revendication 1, **caractérisé en ce que** lesdits moyens de support sont formés par un tampon poreux (48).

3. Dispositif selon la revendication 2, **caractérisé en ce que** ledit corps de drainage (3) comporte des canaux de drainage (39) sous ledit tampon poreux (48), lesdits canaux de drainage débouchant dans une ouverture (38) de sortie.

4. Dispositif selon l'une quelconque des revendications 1 à 3, **caractérisé en ce que** ledit corps de drainage (3) comporte une table circulaire (30) pourvue en son centre desdits moyens de support (48) de ladite membrane (4) et ayant, autour desdits moyens de support (48), une paroi (32) présentant une surface située face audit joint d'étanchéité (13) en élastomère qui forme une partie dudit corps d'admission (2), ladite membrane (4) étant comprimée entre ladite surface (33) et ledit joint d'étanchéité (13).

5. Dispositif selon la revendication 4, **caractérisé en ce que** le diamètre extérieur dudit plateau circulaire (30) correspond sensiblement au diamètre intérieur d'une jupe (6) incorporée dans ledit corps d'admission (2), ladite jupe (6) entourant ladite table circulaire (30).

6. Dispositif selon la revendication 5, **caractérisé en ce que** des surépaisseurs de calage (27) sont prévues entre ladite table circulaire (30) et ladite jupe (6).

7. Dispositif selon l'une quelconque des revendications 4 à 6, **caractérisé en ce que** ledit corps de drainage comporte une jupe (31) disposée en gradin par rapport à ladite table circulaire (30).

8. Dispositif selon la revendication 7, **caractérisé en ce que** ladite jupe (31) comporte des moyens d'encliquetage (42, 44, 45) avec ledit corps d'admission (2).

9. Dispositif selon l'une des revendications 7 ou 8, **caractérisé en ce que** ladite jupe (31) du corps de drainage (3) présente au moins une encoche (47) conçue pour permettre la mise en place d'une seringue de drainage (64).

10. Dispositif selon l'une quelconque des revendications 1 à 9, **caractérisé en ce que** ledit corps de drainage (3) présente un orifice (38) de sortie dans le prolongement du conduit interne d'une tubulure de sortie (37) disposée coaxialement.

11. Dispositif selon la revendication 10, **caractérisé en ce que** ledit corps de drainage (3) comporte, autour de ladite tubulure de sortie (37), une nervure annulaire (40) s'effilant vers son extrémité.

12. Procédé pour drainer un dispositif selon la revendication 11, **caractérisé en ce qu'**on le dispose sur une fiole à vide (71) de façon que ladite tubulure de sortie (37) soit engagée dans le trou central (75) du bouchon (74) de ladite fiole et que ladite nervure annulaire (40) repose sur ce bouchon.
